# EUROPEAN PATENT SPECIFICATION

(11) **EP 0 946 151 B1**
(45) Date of publication and mention of the grant of the patent: **10.05.2006**
(21) Application number: 97947642.1
(22) Date of filing: 12.11.1997
(51) Int. Cl.: A61K 9/22

(54) **ASCENDING-DOSE DOSAGE FORM**
DOSISFORM MIT ANSTEIGENDER DOSISFREISETZUNG
FORME POSOLOGIQUE A DOSES CROISSANTES

(30) Priority: 25.11.1996 US 31741 P; 10.11.1997 US 967606
(43) Date of publication of application: 06.10.1999
(73) Proprietor: ALZA CORPORATION, Palo Alto California 94303-0802 (US)
(72) Inventor: HAMEL, Lawrence, G., Mountain View, CA 94040 (US); AYER, Atul, Devdatt, Palo Alto, CA 94303 (US); WRIGHT, Jeri, D., Dublin, CA 94568 (US); LAM, Andrew, San Francisco, CA 94122 (US); SHIVANAND, Padmaja, Mountain View, CA 94040 (US)
(74) Representative: Golding, Louise Ann
(86) International application number: PCT/US1997/022016
(87) International publication number: WO 1998/023263

(56) References cited:
- WO-A-91/03247
- WO-A-92/01445
- WO-A-92/04012
- WO-A-92/18102
- WO-A-95/19174
- US-A- 4 327 725

## Description

### FIELD OF THE INVENTION

This invention relates to a dosage form for delivering an increasing dose of drug. The invention concerns further a dosage form for delivering an increasing dose of drug per unit time over an extended time for continuous effective therapy. The invention pertains additionally to a novel longitudinal dosage form comprising a lengthwise dimension greater than its diameter-wise dimension for delivering an increased dose of drug over a sustained period of therapy. The invention concerns further a method for delivering a dose of drug from the dosage form provided by this invention in an ascending dose for a known therapeutic purpose.

### BACKGROUND OF THE INVENTION

For a long time, from antiquity to the present, pharmacy and medicine in all societies used medicines as chemotherapeutics, cancer chemotherapeutics, antiviral therapeutics, for treating neurological maladies, as immunosuppressive drugs, for pain relief, for managing mood, thought, feeling, behavior, psychological personality and pharmacological benefits. The medicines used in these therapies are represented by analgesics, antineoplastics, cytoprotectives, vasomodifiers, opioids, barbiturates, hypnotics, central nervous system stimulants, psychostimulants, psychodepressants, alcohols, cannabinoids, catecholamines, and therapies known in the United States Pharmacopeia, 1997 Ed. While these medicines or therapies have a benefit, a serious problem, called tolerance, is associated with their use. The development of tolerance to a drug results from adaptive changes within the affected patient, such that the therapeutic response is reduced in the presence of the same dose of drug. Tolerance to some drugs, for example, to opioids, is characterized by a shortened duration and decreased intensity of the therapeutic effect. Most of the tolerance seen with many drugs is due to adaptation of cells in the nervous system to the drug's in vivo action, as noted in The Pharmacological Basis of Therapeutics, Goodman and Gilman, 7th Ed., p. 534 (1940).

One class of these drugs that has become the standard intervention for the management of behavior and personality, including attention deficit disorders, is the central nervous system stimulants.

The benefits perceived by health-care providers, physicians, psychiatrists, psychologists, social workers, and clinicians are dramatic for central nervous system drugs and this has resulted in the widespread and accepted use of the central nervous system acting drugs to treat attention deficit disorder. In the latest period for collecting data, 1996, it was observed that about two percent of the school-aged female population and about six percent of the school-aged male population, for a total of about two million patients, were administered medication for attention deficit disorder.

Prior to this invention, drugs including opioids, barbiturates, hypnotics, central nervous system stimulants, central nervous system depressants, psychostimulants, alcohols, cannabinoids, catecholamines and other drugs were administered by a standard pharmaceutical dosage form. For example, one prior art dosage form for administering a drug consists in using an immediate release tablet containing the drug. This immediate release form delivers the drug by instant dumping of the drug and produces uneven blood levels characterized by peaks and valleys. For an immediate-release dosage form containing a drug that has a rapid onset and a short half-life, this drug may need multiple doses each day and this can resutt in swings in blood levels as the medication loses its therapeutic effect. This type of dosage form does not provide the needed therapy over an extended time.

Another prior art dosage form for dispensing a drug is the sustained-release dosage form. The sustained-release dosage form delivers a drug in a nonascending profile, often in a descending profile, over time. This dosage form, however, may not provide the required therapy and the appropriate blood pattern. For drugs, such as the central nervous system acting drugs, that are delivered from a sustained-release, nonascending dosage form, the patient often develops an acute tolerance to the drug which is manifested by a short duration and a decrease in the intensity needed for acceptable therapy. The prior art sustained-release dosage form is devoid of means that compensate for its shortcomings inherent therein.

The above presentation teaches that a critical and pressing need exists for a novel dosage form for delivering a drug that overcomes the shortcomings known to the prior art. That is, a long-felt need exists for a dosage form for (1) delivering a drug in a sustained-ascending rate that simultaneously reduces or eliminates the need for frequency of daily dosing; for (2) delivering a drug in a sustained-compensating dose to substantially compensate for acute tolerance to the drug and thereby maintain a preselected clinical profile; for (3) administering the drug in an increasing dose to lessen or eliminate acute or chronic tolerance to the drug to provide effective therapy; and (4) for delivering the drug in a sustained, ascending-controlled profile clinically indicated for both medical and psychomedical effects.

### OBJECTS OF THE INVENTION

Accordingly, in view of the above presentation, it is an immediate object of this invention to provide a novel and unique dosage form that overcomes the shortcomings known to the prior art and thereby makes an advancement in the drug dispensing art.

Another object of the invention is to make available to the medical and mental health arts a novel and unique dosage form that provides an ascending dose of drug over a sustained time.

Another object of the invention is to provide a dosage form for maintaining the therapeutic effect of a drug in a patient that acquires a tolerance to the drug, wherein the dosage form comprises a dose of drug that is released in an ascending dose to a patient that acquired tolerance to the drug to lessen the effect of tolerance and concomitantly provide the intended therapy.

Another object of the invention is to provide a dosage form for maintaining the therapeutic effect of a drug in a patient that acquired acute tolerance to the drug, wherein the dosage form is designed and shaped as a tablet for oral administration and delivers to a patient the drug in a dosage form controlled, increasing dose to compensate for the acute tolerance associated with this drug.

Another object of the invention is to provide a dosage form designed and shaped as an osmotic tablet for oral administration into the gastrointestinal tract, comprising a dose of drug for administering to a patient that acquires chronic tolerance to the drug, for administering the drug in a controlled increasing dose, to provide drug compensation for the acquired chronic tolerance associated with the drug.

Another object of the invention is to make available a dosage form comprising a length greater than its thickness for lessening the incidence of acquired tolerance in a patient, wherein the dosage form is characterized by administering the drug in a sustained and increasing dose overtime to produce the intended therapeutic effect

Another object of the invention is to provide a dosage form manufactured as a longitudinal tablet possessing a lengthwise dimension in excess of its diameter-wise dimension for administering a drug which is a central nervous system stimulant in a controlled, changing dose that overcomes the shortcomings known to the prior art.

Another object of the invention is to make available a method for lessening the incidence of acquired tolerance in a patient administered a drug that develops tolerance in the patient, wherein the method comprises an improvement by administering a dosage form manufactured initially as a compressed tablet that in operation in a fluid environment dispenses a drug in a sustained and increasing dose to substantially lessen the unwanted effects of tolerance and to produce the intended therapy over four hours to thirty hours.

Another object of the invention is to make available a dosage form, designed as an oral pharmaceutically acceptable tablet, comprising a first layer of drug and a second layer that pushes the first layer from the tablet, to provide a sustained and ascending dose of drug.

Another object of the present invention is to make available a method for administering a drug in a sustained and increasing dose by administering an osmotic dosage tablet comprising a drug composition that delivers the drug in an initial concentration, followed by a second, higher concentration from the original drug composition to effect a sustained and ascending dose of drug.

Another object of the invention is to make available a dosage form manufactured as an osmotic tablet comprising a single composition containing a drug administered in a sustained and ascending dose profile over time.

Another object of the invention is to make available a dosage form manufactured as an osmotic tablet comprising an immediate-release dose of drug on the exterior of the osmotic tablet and a prolonged-ascending dose in the interior of the osmotic tablet, which exterior and interior doses operate successively to provide an ascending dose of drug to negate acquired and developed tolerance.

Another object of this invention is to make available a dosage form manufactured as an osmotic oral tablet comprising an immediate dose of drug on the exterior of the osmotic tablet, and an interior bilaminate comprising a drug laminate comprising a dose of drug, and an expandable laminate comprising an expandable hydrogel, which osmotic tablet in operation delivers a dose of drug immediately in up to one hour from the exterior of the osmotic tablet, and a dose of drug in from fifteen minutes up to twenty-four hours from the interior of the osmotic tablet by the expandable laminate pushing the interior drug from the osmotic tablet, whereby through the combined operations of the exterior dose, the interior laminate, and the expandable laminate, the osmotic tablet provides an ascending dose of drug for therapy over time.

These objects, as well as other objects, features and advantages of this invention will become more apparent from the following detailed disclosure of the invention accompanied by the accompanying claims.

### BRIEF DESCRIPTION OF THE DRAWING FIGURES

Figure 1 illustrates an ascending release rate profile for a dosage form comprising the drug pseudoephedrine hydrochloride as provided by the invention;
Figure 2 depicts an ascending-increasing dose release rate for a dosage form of the invention comprising the central nervous system stimulating drug methylphenidate hydrochloride; and,
Figure 3, depicts the release rate in mg/hr for methylphenidate from a dosage form provided by the invention.

### DETAILED DESCRIPTION OF SPECIFICATION

In accordance with the practice of this invention, it has now been discovered a novel dosage form can be made available characterized by an ascending rate of drug delivery over time. The dosage form provided by this invention delivers a drug at a continuously increasing rate for a predetermined period of time. The dosage form of this invention is unexpected and it is a breakaway from the prior art existing dosage form technologies that deliver a drug at a constant zero-order unchanging rate over time. The dosage form of this invention avoids delivery at a zero order rate as it delivers a drug continuously in an ascending rate over time. The profile of the prior art dosage form consists of a short start-up in delivery, followed by a constant unchanged rate. The profile of this invention departs from the prior art by making available a dosage form wherein the drug release rate follows an ascending profile to achieve a desired drug delivery pattern. The dosage form of this invention achieves the ascending pattern by combining the dimensions of the dosage form with the internal formulation of the dosage form.

The dosage form of this invention comprises a wall that surrounds an internal compartment. The wall of the dosage form comprises a semipermeable composition permeable to the passage of fluid present in an environment of use, such as the aqueous biological fluid of the gastrointestinal tract, and the wall is impermeable to the passage of drug. The wall maintains its physical and chemical integrity during the drug dispensing life of the dosage form. The semipermeable wall comprises a polymer selected from the poly(cellulose) group consisting of cellulose acylate, cellulose diacylate, cellulose triacylate, cellulose acetate, cellulose diacetate and cellulose triacetate. The wall comprises 100 wt% (weight percent) of said poly(cellulosic) polymer possessing a number-average molecular weight of 15,000 to 4,000,000. The wall, in another manufacture, can comprise from 40. wt% to 100 wt% of the poly(cellulosic) polymer and from 0 to 35 wt% of a hydroxypropylalkylcellulose selected from the group consisting of hydroxypropylmethylcellulose, hydroxypropylethylcellulose, hydroxypropylbutylcellulose, and hydroxypropylpentylcellulose of 9,000 to 240,000 number-average molecular weight; 0 to 25 wt% of a hydroxyalkylcellulose comprising a member selected from the group consisting of hydroxymethylcellulose, hydroxyethylcellulose, hydroxypropylcellulose, and hydroxybutylcellulose of 7,500 to 200,000 number-average molecular weight, and 0 to 25 wt% of poly(ethylene glycol) of 190 to 40,000 intrinsic viscosity molecular weight. The total weight of all components comprising the wall is equal to 100 wt%. Wall-forming polymers are known in U.S. Patent Nos. 3,845.770; 3,916,899; 4,036,228; 4,111.202; and 5,178,866.

The dosage form comprises one or more than one exit in the wall that connects the exterior of the dosage form with the interior of the dosage form. The term "exit" as used herein comprises a passageway, orifice, pore, micropore, micro-opening, hollow fiber, capillary tube, porous overlay, porous insert, and osmotic opening for dispensing a drug from the dosage form. The exit passageway embraces further a material that erodes, or is leached from the wall in a fluid environment of use, such as the gastrointestinal tract. Representative materials for forming an erodible passageway include erodible poly(glycolic) acid, erodible poly(lactic acid), erodible poly(orthoester), erodible poly(orthocarbonate), erodible poly(acetal), a gelatinous filament, poly(vinyl alcohol), leachable materials including fluid removable pore-forming polysaccharides, salts, sugars and oxides. An exit can be formed by leaching compounds such as sorbitol, lactose or glucose. The exit can have any operative shape such as round, triangular, square, or elliptical. The dosage form can be provided with one or more passageways close together or in spaced apart positions on a common surface of the dosage form. Passageways and equipment for forming an exit are disclosed in U.S. Patent Nos. 3,845,770.- 3,916,899; 4,063,064; 4,088,864; 4,200,098; 4.285.987: and 5,178,866.

The dosage form comprises internally a first drug layer and a second expandable layer. The first layer is next to the exit to provide for drug delivery from the dosage form. The first layer comprises a dose of 10 ng to 700 mg, e.g. 240 ng (nanograms) to 700 mg (milligrams) of drug and up to 200 mg sorbitol. Form 1 mg to 200 mg of a pharmaceutically acceptable carrier may also be present in the first layer. The pharmaceutically acceptable carrier comprises a hydrophilic polymer selected from the group consisting of a poly(alkylene oxide) of 25,000 to 1.000.000 number-average molecular weight where a 5% aqueous solution exhibits a viscosity at 25°C of 12 to 17,600 cps (centipoise) represented by a member selected from the group consisting of poly(methylene oxide), poly(ethylene oxide), poly(propylene oxide), poly(butylene oxide), copolymer poly(ethylene oxide)-poly(propylene oxide); and a blend of two different poly(akylene oxides), such as a poly(alkylene oxide) of 40.000 to 500,000 molecular weight with a different poly(alkylene oxide) of 40.000 to 500.000 molecular weight, a poly(ethylene oxide) of 100,000 molecular weight with a poly(ethylene oxide) of 200.000 molecular weight, or a poly(ethylene oxide) of 200.000 molecular weight with a poly(ethylene oxide) of 300.000 molecular weight, which poly(alkylene oxide) polymers are available from Union Carbide Corp.; 0 mg to 200 mg of a pharmaceutical excipient such as starch, talc, mannitol, glucose, fructose, polysaccharides or silicon dioxide; 0 mg to 125 mg of a hydrophilic pharmaceutically acceptable carboxyvinylpolymer, also known as carboxypolyalkylene polymer, possessing a 7.500 to 1,000,000 number-average molecular weight, including a carboxyvinylpolymer of 450.000 number-average molecular weight, a carboxyvinylpolymer of 750.000 number-average molecular weight, a carboxyvinylpolymer of 1,250,000 number-average molecular weight, and a carboxyvinylpolymer of 3,000,000 number-average molecular weight, as disclosed in U.S. Patent Nos- 2,798,053; 2,909,462; and 3,825,068, and available as Carbopol® polymer from B.F. Goodrich Company; and 0 mg to 250 mg of a pharmaceutically acceptable alkali-carboxyalkylcellulose, wherein the alkali is sodium or potassium represented by sodium carboxymethylcellulose of 10,000 to 7,000,000 viscosity-average number molecular weight, available from the Hercules Corporation; a surfactant selected from 0.0 mg to 7.5 mg of a member selected from the group consisting of amphoteric, anionic, cationic, and nonionic surfactants, as represented by sorbitan trioleate, sorbitan tristearate, ethylene glycol fatty acid ester, polyethylene glycol monostearate, sorbitan sesquioleate, glycerol monostearate, sorbitan monooleate, propylene glycol monolaurate, sorbitan monostearate, diethylene glycol monolaurate, sorbitan monopalmitate, polyoxyethylene mannitol dioleate, sorbitan monolaurate, polyoxyethylene lauryl ether, polyoxyethylene monostearate, polyethylene glycol 400 monostearate, triethanolamine oleate, polyoxyethylene alkyl phenol, polyethylene alkyl aryl ether, potyoxyethytene sorbitan monolaurate, polyoxyethylene sorbitan monostearate, polyoxyethylene sorbitan monooleate, polyoxyethylene sorbitan monopalmitate, polyoxyethylene monostearate, polyoxyethylene sorbitan monolaurate, polyoxyethylene lauryl ether, sodium oleate, and sodium lauryl sulfate, which surfactants are known in Pharmaceutical Sciences, Remington, 17th Ed., pp. 1305-1306 (1985); 0 mg to 20 mg of a hydroxypropylalkylcellulose binder selected from the group consisting of hydroxypropylmethylcellulose, hydroxypropylethylcellulose, hydroxypropylbutylcellulose and hydroxypropylpentylcellulose of 9,000 to 750,000 number-average molecular weight, available from the Dow Chemical Company, and a polyvinylpyrrolidone binder of 7,500 to 350,000 molecular weight; and 0.0 mg to 20 mg of a hydroxyalkylcellulose selected from the group consisting of hydroxymethylcellulose, hydroxyethylcellulose, hydroxypropylcellulose, hydroxybutylcellulose and hydroxypentylcellulose of 7,500 to 750,000 weight-average molecular weight, available from Aqualon Company; and 0.01 mg to 5 mg of a lubricant such as stearic acid. magnesium stearate, calcium stearate, potassium oleate, magnesium laureate and calcium palmitate. The expression "Pharmaceutically acceptable" as used herein means the polymer, compound or drug denotes nontoxic and is acceptable for oral administration to a human patient.

The dosage form comprises a second layer that displaces or pushes the first drug layer through the exit port from the dosage form. The second layer comprises 60 to 95% of a combination of osmopolymer and osmagent. The second layer may also comprise 100 ng to 400 mg of a hydrophilic osmopolymer selected from the group consisting of a poly(alkylene oxide) of 1,500.000 to - 10,000,000 number-average molecular weight; or an alkali carboxyalkylcellulose of 1,750,000 to 10,000,000 of viscosity-average number molecular weight; 0 to 100 mg of a hydroxypropylalkylcellulose comprising hydroxypropylmethylcellulose, hydroxypropylethylcellulose, hydroxypropylbutylcellulose and hydroxypropylpentylcellulose of 9,000 to 750,000 number-average molecular weight 0 to 400 mg, generally 25 mg to 400 mg, of a hydroxyalkylcellulose selected from hydroxymethylcellulose, hydroxyethylcellulose, hydroxypropylcellulose and hydroxybulylcellulose of 7,500 to 1,500,000 number-average molecular weight; up to 250 mg, with a preserit manufacture of 10 mg to 175 mg, of an osmagent, also known as osmotically effective solute, osmotically effective compound, and osmotic agent, including inorganic and organic compounds, selected from the group consisting of magnesium sulfate, magnesium chloride, sodium chloride, lithium chloride, potassium sulfate, sodium sulfate, lithium sutfate, potassium chloride, sodium sulfate, magnesium succinate, tartaric acid; carbohydrates such as raffinose, sucrose, glucose and lactose; from 0.001 mg to 10 mg of a surfactant Selected from the group consisting of amphoteric, anionic, cationic and nonionic surfactants, present in the first drug layer. 0 mg to 20 mg of a carboxyvinylpolymer of 7,500 to 10,000 number-average molecular weight, present in the first drug layer; 0 mg to 5 mg of a colorant compound to identify the dosage form, such as red ferric oxide or black ferric oxide; and 0 mg to 5 mg of a lubricant including the lubricants presented in the first drug layer.

The dosage form provided by this invention is designed and shaped as a dosage form tablet. The dosage form tablet comprises a length greater than its width individually. The dosage form tablet comprises a length of 5 mm to 28 mm, and a width of 2.50 mm to 10 mm. The dosage form tablet provided by this invention comprises in the second layer 60% to 95% of a combination comprising an osmopolymer and an osmagent. The high percent of osmopolymer, or high percent osmopolymer and osmagent composition, joined with the dimensions of the dosage form tablet, enables the dosage form tablet to make available an ascending delivery of drug for 2 hours to 24 hours.

The drug present in the drug layer comprises a drug composition comprising a central nervous system acting drug. Examples of drugs are central nervous system acting drugs for the management of attention deficit disorder, including catecholamines and drugs that can mimic their action. The drugs for this therapy comprise a member selected from the group consisting of amphetamine, dextroamphetamine, methamphetamine, methylphenidate, racemic methylphenidate, threomethytphenidate, ethylphenidate, phenyrisopropylamine and pemoline. The drugs include racemates, stereoisomers and enantiomers of a racemic drug. The drugs include their pharmaceutically-acceptable salts, such as a member selected from the group consisting of hydrochloride, fumarate, sulfate, phosphate, lactate, malate, acetate, tartrate, hydrobromide, citrate, pamoate, maleate, ascorbate, gluconate,-aspartate, and salicylate. WO 91/03247, WO 92/18102, WO 95/19174 and WO 92/04012 disclose drug delivery forms comprising other drugs than the form of the present invention.

The invention may comprise further, on the external surface of the dosage form, a coat composition comprising a drug. The coat composition is an external overcoat carried by the dosage form. The external overcoat on the wall of the dosage form comprises a dose of drug, and the overcoat cooperates with the interior compartment comprising a dose of drug that delivers the drug, to provide an initial unexpected ascending drug delivery profile. The overcoat provides an initial dose of drug followed by a dose of drug from the interior of the dosage form to give an ascending drug delivery profile: The overcoat comprises 10 ng to 100 mg of a drug that is delivered in up to one hour, followed by the dose from the dosage form. The dose of drug from-the interior is delivered over 24 hours. The overcoat comprises a drug which is a central nervous system acting drug. Representative of individual drugs present in the overcoat comprise a drug selected from the group consisting of amphetamine, dextroamphetamine, methamphetamine, methylphenidate, racemic methylphenidate, threomethylphendiate, ethylphenidate, alkylphenidate, phenylisopropylamine, and pemoline. These drugs include their pharmaceutically acceptable salts such as a member selected from the group consisting of hydrochloride, sulfate, phosphate, acetate, hydrobromide, pamoate, malate, maleate, fumarate, ascorbate, tartrate and citrate. Representative of a drug embodiment present in the overcoat is an alkylphenidate comprising 10 ng to 25 mg of methylphenidate.

The overcoat comprises the drug blended with a pharmaceutically acceptable carrier. The pharmaceutically acceptable carrier comprises an aqueous, drug-releasing carrier selected from the group consisting of an alkylcellulose, methylcellulose, ethylcellulose, hydroxyalkylcellulose, hydroxymethylcellulose, hydroxypropylcellulose, hydroxyethylcellulose, hydroxybutylcellulose, hydroxypropylalkylcellulose, hydroxypropylmethylcellulose, hydroxypropylethylcellulose, hydroxypropylbutylcellulose, methyldextrose, acacia, guar gum, pregelatinized starch, propylene glycol alginate and cyclodextrin. The overcoat comprises 0.01 wt% to 15 wt% of the pharmaceutically acceptable carrier. The dosage form overcoat in another manufacture comprises 0.01 wt% to 5 wt% of a member selected from the group consisting of polyethylene glycol, polypropylene glycol, polyvinylpyrrolidone, and acetylated triglycerides. The overcoat provides needed drug therapy, for example, methylphenidate, as the overcoat dissolves or undergoes dissolution in the presence of fluid present in the gastrointestinal tract of a patient. Thus, the overcoat provides drug therapy on oral administration into the patient's drug receiving environment, the gastrointestinal tract, for an immediate therapeutic benefit.

The wall of the dosage form in one manufacture is formed by an air-suspension procedure. This procedure consists of suspending and tumbling compressed bilayer cores in a current of air and wall-forming composition until a wall is applied forming and surrounding an internal compartment containing the bilayer core. The air-suspension procedure is well suited for forming the wall. The air-suspension procedure is described in U.S. Patent Nos. 2,799,241 and 5,082,668. The wall can be formed in an air-suspension coater using cosolvents. Representative cosolvents are: methylene dichloride-methanol, 80:20 wt.wt; or acetone-water cosolvent 85:15. or 90:10, or 95:5, or 99:1 wt.wt, using 1% to 7% solids. Other watt-forming techniques can be used, such as a pan coating system, or a wall forming composition deposited by successive spraying of the composition accompanied by tumbling in a rotating pan. A pan coater may be used to produce thicker walls. A large volume of solvent can be used in a solvent system to produce a thinner wall. Finally, the wall-coated compartment is dried in an oven at 30°C to 50°C for up to a week, or in a humidity controlled oven at 50 RH (relative humidity) and 50°C for 18 hours to 3 days.

The first and second layers of the invention are made by standard manufacturing techniques. For example, in one manufacture the drug and other ingredients are blended and pressed into a solid layer. The drug and the ingredients can be blended with a solvent and mixed into a semisolid or solid formed by conventional methods such as ball-milling, calendering, stirring or roller milling, and then pressed into a preselected shape. The first layer possesses dimensions that correspond to the internal dimensions of the area the layer occupies in the dosage form. It also possesses dimensions corresponding to the second layer for forming a contacting bilayer arrangement therewith. The push layer comprising the osmopolymer and osmagent, is placed in contact with the first drug layer. The push layer a displacement layer for displacing the drug layer from the dosage form is manufactured using the techniques for providing the first drug layer. The layering of the first drug layer and the second displacement layer can be fabricated by conventional press layering techniques. The bilayered compartment-forming core is surrounded and coated with an outer wall comprising a semipermeable composition. An exit is laser drilled through the wall to contact the first drug layer. The dosage form is optically oriented automatically by the laser equipment for forming the exit passageway.

In another manufacture, the dosage form is manufactured by the wet granulation technique. In the wet granulation technique, for example, the drug and the ingredients comprising a drug layer are blended using an organic solvent, such as isopropyl alcohol-methylene dichloride 80:20 v:v (volume:volume), or methanol-methylene dichloride, as the granulation fluid. Other granulating fluid, such as denatured alcohol 100%, can be used for this purpose. The ingredients forming the drug layer are individually passed through a screen and then thoroughly blended in a mixer. Next, other ingredients comprising the drug layer are dissolved in a portion of the granulation fluid, such as the solvents described above. Then, the latter prepared wet blend is slowly added to the drug blend with continual mixing in the blender. The granulating fluid is added until a wet blend is produced, which wet mass is forced through a screen onto oven trays. The blend is dried for 7 to 24 hours at 30°C to 50°C. The dry granules are then sized with a screen. -Next, a lubricant is passed through a screen and added to the dry screen granule blend. The granulation is put into milling jars and mixed on a jar mill for 1 to 15 minutes. The other drug layer and the displacement layers are made by the same wet granulation techniques. The compositions are pressed into their individual layers in standard presses, such as a layer press.

Another manufacturing process that can be used for providing the compartment-forming compositional layers comprises blending the powdered ingredients for each layer independently in a fluid bed granulator. After the powdered ingredients are dry blended in the granulator, a granulating fluid, for example, poly(vinylpyrrolidone) in water, or in denatured alcohol, or in 95:5 ethyl alcohol/water, or blends of ethanol and water, is sprayed onto the powders. Optionally, the ingredients can be dissolved or suspended in the granulating fluid. The coated powders are then dried in a granulator. This process granulates all the ingredients present therein while adding the granulating fluid. After the granules are dried, a lubricant, such as stearic acid, calcium stearate, magnesium oleate, potassium oleate, or magnesium stearate, is added to the granulator. The granules for each separate layer are then pressed in the manner described above.

The dosage form of this invention is manufactured in another manufacture by first mixing a known dose of drug with compositional layer-forming ingredients, and then pressing under one-eighth to three tons the composition into a solid layer possessing dimensions that correspond to the internal dimensions of the compartment of the dosage form. The contacting second layer, comprising an osmopolymer and an osmagent, is manufactured in a like manner. In another manufacture, the first and second layers independently are manufactured by mixing the drug and composition forming ingredients and a solvent into a solid, or into a semisolid, by conventional methods such as ball milling, calendering, stirring or roller milling, and pressed into a layer. Next, the first layer is placed next to a layer of a displacement composition comprising an osmopolymer and an osmagent. Then, the two-layered core is surrounded with a semipermeable wall. The layering of the first layer and the second layer can be accomplished by layer tablet press technique and longitudinal compressed tablet technique-A wall can be applied by molding, spraying or dipping the pressed-shape bilayered core into wall forming materials. Another technique that can be used for applying the wall is the air-suspension coating procedure. This procedure consists in suspending and tumbling the two-layered laminate in a current of air until the wall forming composition surrounds the bilayer. The air-suspension procedure is described in U.S. Patent No. 2,799,241: J. Am. Pharm. Assoc., Vol. 48, pp. 451-459 (1979); and ibid., Vol. 49, pp. 83-84 (1960). Other manufacturing procedures are described in Modern Plastic Encyclopedia, Vol. 46, pp. 62-70 (1969): and in Pharmaceutical Sciences, Remington, 14th Ed., pp. 1626-1979 (1970), published by Mack Publishing Co., Easton, PA.

Exemplary solvents suitable for manufacturing the wall the layer and the wall include inert inorganic and organic solvents. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane ethylene glycol monoethyl ether, ethylene glycol monoethyl acetate, methylene dichloride, ethylene dichloride, propylene dichloride, carbon tetrachloride, chloroform, nitroethane, nitropropane, tetrachloroethane, ethyl ether, isopropyl ether, cyclohexane, cyclooctane, benzene, toluene, naphtha, tetrahydrofuran, diglyme, aqueous and nonaqueous mixtures thereof, such as acetone and water, acetone and methanol, acetone and ethyl alcohol, methylene dichloride and methanol, ethylene dichloride and methanol, and methylene dichloride and ethanol. The solvents are disdosed in U.S- Patent No. 5,030,456.

### DETAILED DESCRIPTION OF THE EXAMPLES

The following examples are illustrative of the present invention. Examples 1-10 do not fall within the scope of the invention as claimed. The techniques described therein are nevertheless applicable to the preparation of the dosage form of the invention.

### EXAMPLE 1

An osmotic dosage form designed and shaped to deliver a drug that stimulates the central nervous system by administering a drug in an ascending release profile is manufactured as follows: First, a binder solution is prepared as follows: 300 g of poly(vinylpyrrolidone) having a number-average molecular weight of 40.000 is added to a mixing vessel containing 2700 g of distilled water.- The mixture is stirred until the poly(vinylpyrrolidone) dissolves in the water and produces a clear solution. Next, a drug formulation is prepared as follows: 6,564 g of poly(ethylene oxide) having a number-average molecular weight of 200,000 is passed through a 40 mesh screen. Then, 3,282 g of the screened poly(ethylene oxide) is placed into the bowl of a fluid bed granulator. Next, 1,024 g of central, nervous system acting methylphenidate hydrochloride is placed into the granulator with the poly(ethylene oxide). Then, 100 g poly(vinylpyrrolidone) of number-average molecular weight 40,000 is added to the granulator; then the remaining 3,182 g of poly(ethylene oxide) is added to the granulator. The addition of the dry ingredients into the bowl is performed so that the methylphenidate hydrochloride is located between two layers of poly(ethylene oxide). The granulation is started with the ingredients inside the bowl fluidized for 2 minutes to obtain a uniform mixing of the powders. Next, the binder solution is sprayed onto the powder bed through nozzles at a spray rate of 100 g/min. During the spraying process, the airflow is maintained at 500 cfm and the temperature kept at 25°C. During the spraying operation, the solution is sprayed for 30 seconds, followed by a shaking time of 10 seconds during which time the powders adhering to the filterbags are dislodged into the granulating chamber. At the end of the spraying operation, the granules are dried in the granulating chamber for an additional 5 to 10 minutes to obtain dry granulation. The methylphenidate hydrochloride granules are passed through a fluid air mill with a 7 mesh screen for size reduction. The screen is U.S. Sieve Series as disclosed in Chemical Engineer's Handbook, Perry, 6th Ed., pp. 21-15 (1984). Next, the screened granules are placed in a blender to which 8 g of magnesium stearate (screened through a 40 mesh screen) and 4 g of powdered butylated hydroxy toluene (screened through a 60 mesh screen) is added to the granules and mixed together.

Next, the displacement or push composition is prepared as follows: First a binder solution is prepared by adding 937.5 g of poly(vinylpyrrolidone) of 40,000 number-average molecular weight to 8437.5 g of distilled water. This mixture is stirred until the poly(vinylpyrrolidone) dissolves in water to yield a clear solution. Next, displacement osmotic granules are prepared as follows: First, 13452.5 g of poly(ethylene oxide) having a number-average molecular weight of 7,000,000 is placed into the bowl of a fluid bed granulator. Next, 312.5 g of poly(vinylpyrrolidone) of 40,000 number-average molecular weight is added to the bowl. Then, 10,000 g of osmagent sodium chloride and 250 g of red ferric oxide, which have been milled using a 21 mesh screen, are placed into the bowl. The bowl is attached to the main body of the granulator and the granulation is initiated. Initially, the powder bed inside the bowl is fluidized for 3 minutes to obtain uniform mixing of the powders. Then, the binder solution is sprayed onto the powder bed and the binder solution sprayed thereon at a rate of 240 g/min. During the spraying, the air flow is maintained at 1000 cfm and the temperature kept at 25°C. The solution is sprayed for 30 seconds, followed by shaking for 10 seconds, during which time the powders adhering to the filterbags can be dislodged into the granulating chamber. At the end of the spraying operation, the granules are dried in the granulating chamber for an additional 10 to 15 minutes to obtain dry granulation. The granules are then passed through an air mill with a 7 mesh screen for size reduction. The granules are then transferred to a blender, and 25 g of magnesium stearate (screened through a 40 mesh screen) and 12.5 g of butylated hydroxy toluene (screened through a 60 mesh screen) are added to the granules and mixed together.

Next, the drug composition comprising the methylphenidate and the displacement osmotic composition are compressed together using an automated tablet compression machine capable of compressing the two layers longitudinally together. First, 110 mg of the methylphenidate composition forming layer is added to the die cavity of 4.7 mm diameter, tamped, and then 132 mg of the displacement osmotic composition is placed into the die and compressed together using 0.2 metric tons of pressure.

Next, a wall-forming composition comprising 90% cellulose acetate having an acetyl content of 39.8%, and 10% poly(ethylene glycol) having an average molecular weight of 3350 is formed around the bilayered core. The semipermeable composition is dissolved in a mixture of acetone and water (90:10 wt:wt) to provide solid composition of the solution at 5%. The compressed bilayered cores are placed into a 61 cm coating pan and the coating solution sprayed onto the cores at a spray rate of 100 ml/min. The temperature is kept at 35°C during the coating process.

Next, one 30 mil (0.76 mm) orifice is drilled through the semipermeable wall connecting the drug composition with the exterior of the dosage form. The residual solvent is removed by drying at 45°C and 45% relative humidity in an oven for 48 hours. At the end of the drying cycle, the humidity is turned off, and the dosage forms are dried at 45°C for an additional 4 hours.

The dosage form prepared according to this example comprises a first drug layer comprising 110 mg consisting of 14.08 mg of methylphenidate, 90.26 mg of poly(ethylene oxide) of 200,000 number-average molecular weight, 5.5 mg of poly(vinylpyrrolidone) of 40,000 number-average molecular weight, 0.11 mg of magnesium stearate, and 0.055 mg of butylated hydroxy toluene. The dosage form displacement layer 2 comprises 132 mg composed of 71.032 mg of poly(ethylene oxide) of 7,000,000 number-average molecular weight, 52.8 mg of osmagent sodium chloride, 6.6 mg of poly(vinylpyrrolidone) of 40,000 number-average molecular weight, 1.32 mg of red ferric oxide, 0.132 mg of magnesium stearate, and 0.066 mg of butylated hydroxy toluene. The semipermeable wall weighed 17 mg and comprised 15.3 mg of cellulose acetate consisting of 39.8% acetyl content, and 1.7 mg of poly(ethylene glycol) of 3350 number-average molecular weight. The dosage form possessed a 30 mil (0.76 mm) orifice.

The dosage form delivered methylphenidate in an ascending release rate. The dosage from delivered 0.22 mg in the first hour, 1.45 mg in the second hour, 1.72 mg in the third hour, 1.84 mg in the fourth hour, 2.05 mg in the fifth hour, 2.21 mg in the sixth hour, 2.13 mg in the seventh hour, 1.26 mg in the eighth hour, 0.39 mg in the ninth hour, and 0.09 mg in the tenth hour, with a residual of 0.72 mg in the dosage form.

### EXAMPLE 2

The procedure of Example 1 is followed with the processing steps as previously described, except the drug in this example is a member selected from the group consisting of pemoline, deanol, deanol acetamidobenzoate, benzphetamine hydrochloride, deanol aceglumate, clortermine, diethylpropion, fenfluramine, dextroamphetamine phosphate, and dextroamphetamine sulfate.

### EXAMPLE 3

An osmotic dosage form adapted, designed and shaped as an oral tablet for delivering a drug with an ascending release profile is manufactured as follows: First, a composition is prepared by passing through a 40 mesh screen 393.85 g of poly(ethylene oxide) having a molecular weight of about 300,000. Then 63.65 g of drug selected from the group consisting of amphetamine sulfate, difluanine hydrochloride, flubanilate, mefexamide, methamphetamine, pseudoephedrine and pyrovalerone hydrochloride; 15.00 g of polyoxyl 40 stearate; and 25.00 g of hydroxypropylmethylcellulose having a number-average molecular weight of 11,200 are screened to 40 mesh and then added to the poly(ethylene oxide). The four ingredients are mixed for about 10 minutes in a conventional mixer. Then 50 ml of denatured, anhydrous ethanol is slowly added to the mixer and the mixing continued for an additional 10 minutes. The wet granulation is passed through a 20 mesh screen, dried at room temperature for 16 hours, and passed again through a 20 mesh screen. Finally, 2.5 g of magnesium stearate is added to the granulation and all the ingredients mixed for an additional 3 minutes.

Next, a second displacement composition is prepared by mixing 267.5 g of poly(ethylene oxide) having a number-average molecular weight of 7,000,000 with 175 g (35%) of osmagent sodium chloride; 25 g of Carbomer® 934, a carboxyvinyl polymer possessing a 3,000,000 number-average molecular weight, disclosed in U.S. Patent Nos. 3,074,852, 3,634,584, and 4,248,847, commercially available from BF Goodrich Chemicals, Cleveland, OH; and 5 g of red iron oxide. The homogenous blend is passed through a 40 mesh screen, and 25 g of hydroxypropylmethylcellulose having a number-average molecular weight of 11,200 is added to all the ingredients.- The ingredients are blended in a conventional planetary mixer for 10 minutes. Then, 50 ml of denatured, anhydrous ethanol is slowly added to the blending mixture and all the ingredients mixed for an additional 5 minutes. The freshly prepared wet granulation is passed through a 20 mesh screen, allowed to dry at room temperature for 16 hours, and again passed through a 20 mesh screen. The screened granulation is mixed with 2.5 g of magnesium stearate for 3 minutes.

A laminating press is used to form the bilaminate. First, 110 mg of the first drug composition is added to a 4.7 mm die cavity and tamped. Then, 115 mg of the second expandable composition is added to the die cavity, and the two separate compositions pressed into a bilaminated core under 1/2 ton of pressure.

Next, the bilartlinated core is surrounded with a semipermeable wall. The wall-forming composition comprises 95% cellulose acetate having an acetyl content of 39.8%, and 5% polyethylene glycol having a molecular weight of 3350. The wall forming composition is dissolved in acetone:water (90:10 wt:wt) solvent to make a 5% solids solution. Then, 22 mg of the wall forming composition is sprayed onto and around this bilaminate in a Hi-Coater pan coater. Then, a 30 mil (0.76 mm) exit orifice is drilled by laser or mechanical drill in the center of the drug laminate side of the osmotic device. The residual solvent is removed by drying for 48 hours at 50°C and 50% relative humidity, followed by one hour drying at 50°C.

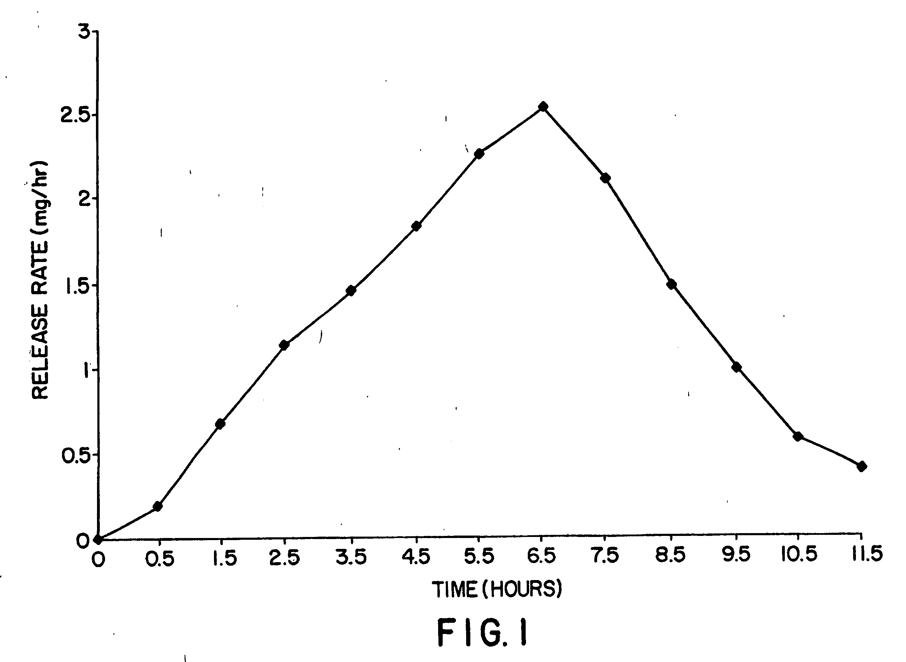

A dosage form prepared by this example comprises a 110 mg drug layer comprising 12.73 wt% pseudoephedrine hydrochloride, 78.77 wt% poly(ethylene oxide) of 300,000 number-average molecular weight, 5 wt% hydroxypropylmethylcellulose of 11,200 number-average molecular weight, 3 wt% polyoxyl 40 stearate and 0.5 wt% magnesium stearate. The 115 mg displacement layer comprises 53.5 wt% poly(ethylene oxide) possessing a 7,000,000 number-average molecular weight, 5 wt% hydroxypropylmethylcellulose possessing 11,200 number-average molecular weight, 35 wt% sodium chloride, 0.5 wt% magnesium stearate, 5 wt% Carbopol® carboxyvinylpolymer, and 1 wt% red ferric oxide. The semipermeable wall comprises 20.9 mg of cellulose acetate of 39.8% acetyl content and 1.1 mg of poly(ethylene glycol) of 4,000 number-average molecular weight. The dosage form comprises a 30 mil orifice (0.76 mm) and exhibits the ascending release rate profile seen in accompanying Figure 1.

### EXAMPLE 4

A dosage form designed and adapted as a tablet for the oral administration of methylphenidate pharmaceutically acceptable salt in an ascending release profile is manufactured as follows: First, 163.4 g of poly(ethylene oxide) having an average molecular weight of 200,000 is passed through a 40 mesh screen and placed into the bowl of a conventional planetary mixer. Next, 25.6 g of methylphenidate hydrochloride is weighed and placed into the bowl containing the poly(ethylene oxide). Next, 10 g of hydroxypropylmethylcellulose possessing a 11,200 molecular weight is passed through a 40 mesh screen and placed into the bowl containing the poly(ethylene oxide) and methylphenidate hydrochloride. Next, 0.5 g of FD&C Blue Dye No. 1 is placed into the bowl of the mixer. The four ingredients are blended together in the planetary mixer for 10 minutes. Next, 100 ml of denatured anhydrous ethanol is gradually added to the mixer with continued mixing for 10 minutes to change the consistency of the dry powder to that of granules. The wet granulation is then passed through a 20 mesh screen, dried at room temperature for 16 hours, and then passed through a 20 mesh screen. Next, 0.5 g of magnesium stearate, which has been passed through a 40 mesh screen, is added to the granulation, and all the ingredients are mixed for an additional 1 minute.

Next, a displacement layer is manufactured as follows: First, 107 g of poly(ethylene oxide) having an average molecular weight of 7,000,000, 80 g of sodium chloride (40%), 10 g of hydroxypropylmethylcellulose (USP grade) possessing a 11,200 molecular weight, and 2 g of red ferric oxide are passed through a 40 mesh screen and then placed into the bowl of a conventional planetary mixer. The powder mixture is then blended together until a homogenous blend is formed. Next, 50 ml of denatured anhydrous ethanol is added to the mixer with continued mixing over a period of 5 to 10 minutes, such that the consistency of the dry powder changes to granules. The wet granulation is passed through a 20 mesh screen, dried at room temperature for 16 hours, and then passed through a 20 mesh screen. Next, 1 g of magnesium stearate which has been passed through a 40 mesh screen is added to the granulation, and all the ingredients are mixed for an additional 1 minute.

Next, a bilayer press is used to compress the two layers together to form a tablet dosage form. First, 110 mg of the drug composition is added to the 4.7 mm die cavity and lightly tamped. Next, 115 mg of the displacement composition is weighed and placed into the die cavity, and the two layers are compressed together using 1/2 ton of pressure to form a bilayer pretablet.

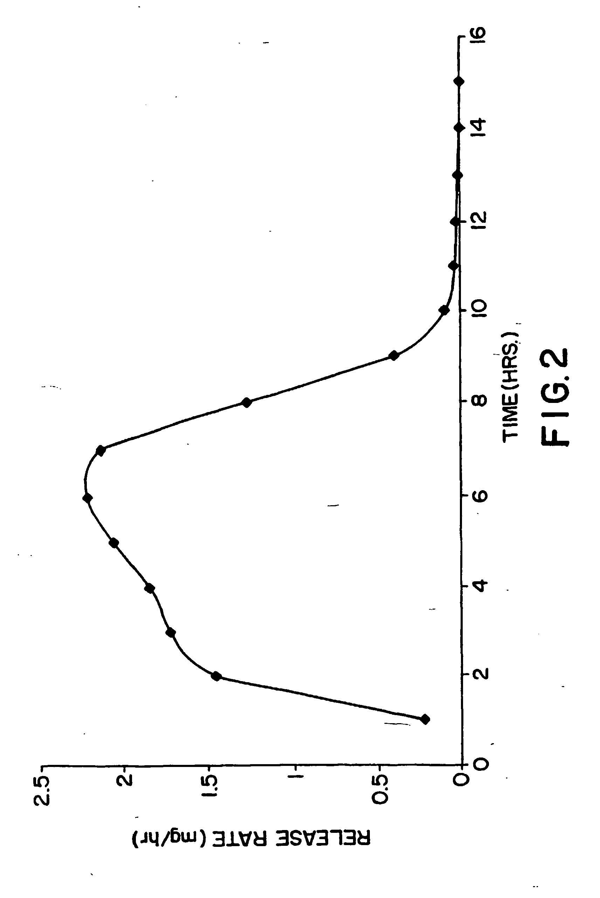

A wall for enveloping the bilayer pretablet to yield the finished tablet is formed as follows: First, a semipermeable composition composed of 90% cellulose acetate (having an acetyl content of 39.8%) and 10% polyethylene glycol having an average molecular weight of 3350 is prepared by dissolving in a mixture of acetone and water (the solvents are mixed together in a ratio of 90:10 wt:wt), such that the solids composition of the solution is 5%. The bilayer tablets are placed in a coater pan, and 20 mg of the semipermeable composition is sprayed on to the bilayer tablets. Next, a 30 mil (0.76 mm) orifice is drilled on the drug layer side of the bilayer tablet using an automatic tableting positioning laser drill. Next, the semipermeable coated tablets are dried for 48 hours at 50°C and 50% relative humidity to remove the residual solvents. The release rate ascending profile for a dosage form prepared according to the example is seen in accompanying Figure 2. Drawing Figure 2 depicts the release of methylphenidate hydrochloride from a semipermeable, longitudinal dosage form.

### EXAMPLE 5

The procedure of the above examples is followed, with the further embodiment that a drug overcoat and an optional taste-masking coat is overcoated onto the exterior surface of the wall. The overcoat, in one manufacture, comprises 60 wt% of hydroxypropylmethylcellulose of 9,200 number-average molecular weight-and 40 wt% methylphenidate hydrochloride. The hydroxypropylmethylcellulose is added to water and mixed until a uniform solution results. Then, the methylphenidate hydrochloride is added to the solution and mixed such that a clear solution results. The final solution has a solid composition of 10%. Next, semipermeable walled dosage forms are placed in a coater and 10 mg of the drug overcoat is sprayed onto the semipermeable wall that surrounds the internal bilayer compressed tablet. Then, overcoated dosage forms are dried for 10 minutes at 40°C. For taste masking, a suspension of Opadry® , a powder blend comprised of hydroxypropylmethylcellulose, titanium dioxide, polyethylene glycol and polysorbate 80, is prepared in water to effect a solid content of 10%. The drug overcoated dosage forms are placed in a coater and 9 mg of the taste-masking solution is sprayed over the drug overcoat to produce a double-overcoated dosage form. Next, the dosage forms are dried at 40°C for 10 to 12 minutes to yield the operable dosage forms.

### EXAMPLE 6

The procedures set forth in the above examples are followed, with the manufactures as described above, except in this invention, a first dosage form is provided wherein the drug layer comprises 28 mg of methylphenidate hydrochloride, and a second dosage form is provided wherein the drug layer comprises 42 mg of methylphenidate hydrochloride.

### EXAMPLE 7

The procedures set forth in the above examples are followed with the manufacturing conditions set forth as previously indicated, except that in this example a dosage form is provided wherein the second displacement layer comprises 65 mg of sodium carboxymethylcellulose osmopolymer of 3,500,000 number-average molecular weight and an osmagent combination comprising 58 mg of dextrose-fructose in equal proportions; and a dosage form wherein the second displacement layer comprises 72 mg of poly(ethylene oxide)-poly(propylene oxide) copolymer of 7,900,000 number-average molecular weight and 47.8 mg of co-osmagent sodium chloride-dextrose (23.9 mg - 23.9 mg).

### EXAMPLE 8

A dosage form designed and adapted to deliver a drug in an ascending release rate profile is manufactured according to this example.

First, a first layer-forming composition comprising a dose of drug is manufactured as follows: 157.8 mg of poly(ethylene oxide) having a number-average molecular weight of 200,000 is passed through a 40 mesh screen (U.S. Sieve) and placed into the bowl of a conventional planetary mixer. Next, 31.2 g of the drug methylamphetamine hydrochloride is added to the mixer. Then, 10 g of hydroxypropylmethylcellulose of 16,000 number-average molecular weight is passed through a 40 mesh screen and added to the mixer comprising the methylamphetamine hydrochloride and the poly(ethylene oxide). Then, 0.5 g of FD&C Blue Dye No. 1, for color identification, is added to the bowl of the mixer. The ingredients are blended in the mixer for 10 minutes to produce a homogenous composition. Next, 100 ml of denatured anhydrous ethanol is added gradually to the mixer, with continual mixing over a period of 5 to 10 minutes to change the consistency of the dry ingredients to wet granules. The wet granulation is passed through a 20 mesh screen, dried at room temperature for 16 hours, and then passed through a 20 mesh screen. Then, 0.5 g of magnesium palmitate is passed through a 40 mesh screen, added to the homogenous composition, and all the ingredients mixed for an additional minute.

Next, a displacement layer, the second layer, is prepared as follows: First, 53 g of poly(ethylene oxide) of 7,500,000 number-average molecular weight, 54 g of poly(propylene oxide) of 5,000,000 number-average molecular weight, 80 g of osmagent sodium chloride, 10 g of hydroxypropylethylcellulose of 24,000 number-average molecular weight, and 2 g of red ferric oxide are passed through a 40 mesh screen and then placed into the bowl of a mixer. The ingredients are blended together to form a homogenous blend. Next, 50 ml of denatured anhydrous ethanol is added to the mixer, accompanied by continual mixing for 10 minutes to produce wet granules. The wet granules are passed through a 20 mesh screen, dried at room temperature for 16 hours, and then passed through a 20 mesh screen. Next, 1 g of stearic acid lubricant is passed through a 40 mesh screen, added to the granulation, and all the ingredients mixed for an additional minute.

Next, the first drug layer-forming composition and the second displacement layer-forming composition are pressed together into contacting layers as follows: First, 33 mg of the first composition is added to a 0.55 cm die cavity and tamped lightly. Then, 57 mg of the displacement composition is added to the die and tamped lightly, to provide two layers that are compressed using 1/2 ton of pressure to form a bilayer tablet.

Next, the bilayer is surrounded with a semipermeable wall as follows: First, a semipermeable wall forming composition is prepared comprising 95% cellulose having an acetyl content of 39.8%, and 5% polyethylene glycol of 3350 number-average molecular weight (available from Union Carbide Co.) by dissolving the ingredients in a mixture of acetone and water in a 90:10 (v:v) ratio to provide solid composition at 5%. The bilayer tablets are placed in a pan coater and 15 mg of the semipermeable wall forming composition is sprayed onto the bilayer tablet. Next, a 30 mil (0.76 mm) orifice is drilled on the drug side to connect the first layer with the outside of the dosage form. The dosage form tablets are dried at 50°C and 50% relative humidity to remove the residual solvents. The dosage form comprises 5.2 mg of methamphetamine hydrochloride to give an extended ascending release tablet. Additional dosage forms are provided according to the invention comprising 10 mg and 15 mg methamphetamine hydrochloride extended ascending release tablet.

### EXAMPLE 9

The above examples are followed to provide a dosage form comprising an external coat of a pharmaceutically acceptable drug and an internal composition comprising a pharmaceutically acceptable drug, which dosage form when in operation in a fluid biological environment delivers the external overcoat drug in 0 to 1 hour, and delivers the internal drug in the amounts of 15% in 0 to 2 hours, 30% in 2 to 4 hours, 33% in 4 to 6 hours, 18% in 6 to 8 hours, and 4% in 8 to 10 hours.

### EXAMPLE 10

The above examples are followed to provide a dosage from comprising an external overcoat comprising 100 ng to 100 mg of drug and an internal composition comprising 10 ng to 500 mg of drug, which dosage form when in operation in the fluid environment of the gastrointestinal tract delivers the 100 ng to 100 mg external overcoat in 0 to 1 hour, and delivers the internal 10 ng to 500 mg in an increasing release of 20% in 0 to 4 hours, 30% in 4 to 8 hours, 40% in 8 to 12 hours and 10% in 12 to 16 hours, to substantially overcome tolerance to delivered drug.

### EXAMPLE 11

An osmotic dosage form designed and shaped to deliver methylphenidate hydrochloride to a patient in need of methylphenidate therapy in an ascending release profile is manufactured as follows:

Composition of drug layer 1: The following procedure is used to manufacture 8,000 g of layer 1 composition:

### A. Preparation of binder solution.

260 g of hydroxypropylmethylcellulose having average molecular weight of 11,200 is added to a mixing vessel containing 3250 g of water. This mixture is stirred until the hydroxypropylmethylcellulose dissolves in water and a clear solution is formed. This solution is referred to as the binder solution.

### B. Preparation of methylphenidate hydrochloride granules.

4380 g of polyethylene oxide having an average molecular weight of 200,000 is passed through a 40 mesh screen. Then, 2190 g of the screened poly(ethylene oxide) is placed into the bowl of a fluid bed granulator. Next, 2032 g of sorbitol is added to the powder bed, followed by 1024 g of methylphenidate hydrochloride into the bowl over the poly(ethylene oxide). Next, 140 g of hydroxypropylmethylcellulose is added to the bowl. The remaining 2190 g of poly(ethylene oxide) is then added to the bowl. The addition of dry ingredients into the bowl is performed so that the methylphenidate hydrochloride is located in between the two layers of poly(ethylene oxide). The bowl is attached to the main body of the granulator and the granulation process is initiated. Initially, the powder bed inside the bowl is fluidized for 2 minutes to obtain uniform mixing of the powders. Next, the binder solution is sprayed onto the powder bed through nozzles such that the solution is sprayed at a rate of 60 g/min. During the spraying process, the process air flow is maintained at 500 cfm and the product temperature is maintained at 22°C. During the spraying operation, the solution is sprayed for 30 seconds followed by a shaking time of 10 seconds, during which time the powders adhering to the filterbags may be dislodged into the granulating chamber. At the end of the spraying operation, the granules are dried in the granulating chamber for an additional 5 to 10 minutes to obtain dry granulation. The methylphenidate hydrochloride granules are then passed through a fluid air mill with a 7 mesh screen for size reduction. The size-reduced granules are then placed into a suitable blender. 160 g of magnesium stearate (screened through a 40 mesh screen) and 4 g of powdered butylated hydroxy toluene (screened through a 60 mesh screen) are added to the granules and mixed together.
Composition of layer 2: The following procedure is used to manufacture 8,000 g of displacement layer 2 composition:

### A. Preparation of binder solution.

260 g of hydroxypropylmethylcellulose having an average molecular weight of 11,200 is added to a mixing vessel containing 3250 g of water. This mixture is stirred until the hydroxypropylmethylcellulose dissolves in water and a clear solution is formed. This solution is referred to as the binder solution.

### B. Preparation of osmotic layer granules.

4308 g of poly(ethylene oxide) having an average molecular weight of 7,000,000 is placed into the bowl of a fluid bed granulator. Next, 140 g of hydroxypropylmethylcellulose having an average molecular weight of 11.200 is added to the bowl. Then, 3,200 g of sodium chloride and 80 g of red ferric oxide, which have been screened using a 21 mesh screen, are then placed into the bowl. The bowl is attached to the, main body of the granulator and the granulation process is then initiated. Initially, the powder bed inside the bowl is fluidized for 3 minutes to obtain uniform mixing of the powders. Next, the binder solution is sprayed onto the powder bed through nozzles, such that the solution is sprayed at a rate of 80 g/min. During the spraying process, the process air flow is maintained at 400 cfm, and_the product temperature is maintained at 22°C. During the spraying operation, the solution is sprayed for 30 seconds followed by a shaking time of 10 seconds, during which time the powders adhering to the filterbags may be dislodged into the granulating chamber. At the end of the spraying operation, the granules are dried in the granulating chamber for an additional 10 to 15 minutes to obtain dry granulation. The process parameters may be adjusted to obtain a quality product. The granules then are passed through a fluid air mill with a 7 mesh screen for size reduction. The size-reduced granules are then placed into a suitable blender. Next, 8 g of magnesium stearate (screened through a 40 mesh screen) and 4 g of powdered butylated hydroxy toluene (screened through a 60 mesh screen) are added to the granules and mixed together.

### C. Compression of the layers.

The methylphenidate granules-forming layer and the osmotic granules-forming layer are compressed together using an automated tablet compression machine capable of compressing the two layers together longitudinally. First, 110 mg of methylphenidate granules (layer 1) is added into the die cavity of a 4.76 mm (3/16") diameter modified ball tooling, tamped, and then 132 mg of the osmotic layer granulation (layer 2) is placed into the die and compressed together using 0.2 metric tons of pressure.

### D. Application of semipermeable membrane wall.

The semipermeable membrane wall is composed of 47.5% cellulose acetate (having an acetyl content of 39.8), 47.5% cellulose acetate (having an acetyl content of 32.0), and 5% polyethylene glycol having an average molecular weight of 3350. The semipermeable membrane-forming composition is dissolved in a mixture of methylene chloride and methanol (the solvents are mixed together in a ratio of 80:20 wt:wt), such that the solids composition of the solution is 4%. The compressed systems are placed into a 61 cm coating pan and the coating solution is sprayed onto the tablets such that the solution is sprayed at a rate of 100 ml/min/gun. The product temperature is maintained at 25°C; the coating process is stopped when the semipermeable membrane composition has been sprayed onto the compressed systems.

Next, one 30 mil (0.76 mm) orifice is drilled, using a mechanical drill bit or a laser, on the drug layer side of the coated systems. The residual solvents remaining after the coating are removed by drying the systems at 45°C and. 45% relative humidity in an oven for 48 hours. At the end of this drying cycle, the humidity is turned off, and the systems are dried at 45°C for an additional 4 hours to complete the drying process.

A methylphenidate dosage form assembled as described contains 110 mg of drug containing layer 1, which is composed of 12.8% methylphenidate hydrochloride, 54.75% poly(ethylene oxide) of average molecular weight 200,000, 25.4% sorbitol, 5% hydroxypropylmethylcellulose of average molecular weight 11,200, 2% magnesium stearate and 0.05% butylated hydroxy toluene. The dosage form also contains 132 mg of layer 2, composed of 53.85% poly(ethylene oxide) of average molecular weight 7,000,000, 40% sodium chloride, 5% hydroxypropylmethylcellulose of average molecular weight 11,200, 1% red ferric oxide, 0.1 % magnesium stearate and 0.05% butylated hydroxy toluene. The 42 mg semipermeable laminate is composed of 47.5% cellulose acetate of acetyl content 39.8% and 47.5% cellulose acetate of acetyl content 32.0%, and 5% polyethylene glycol having an average molecular weight of 3350 is applied to the compressed bilayer system. A 30 mil (0.76 mm) orifice is drilled on the drug layer side as the exit orifice. The final system is capable of delivering 14 mg of methylphenidate hydrochloride with an ascending release rate profile over time.

The dosage form delivered methylphenidate hydrochloride in an ascending rate. The dosage form delivered 0.13 mg in the first hour, 1.16 mg in the second hour, 1.53 mg in the third hour, 1.61 mg in the fourth hour, 1.75 mg in the fifth hour, 1.79 mg in the sixth hour, 2.13 mg in the seventh hour, 2.18 mg in the eighth hour, 1.07 mg in the ninth hour, and 0.43 mg in the tenth hour, 0.17 mg in the eleventh hour, and 0.13 mg in the twelfth hour.

### EXAMPLE 12

An osmotic dosage form designed, shaped and adapted for delivering methylphenidate pharmaceutically acceptable salt to a patient in need of methylphenidate therapy in an ascending release rate profile is manufactured as follows: First, the procedure used to manufacture the drug layer granulation in Example 11 is followed in this example. The composition of the drug layer can comprise from 0% to 30% sorbitol. Next, the following procedure is used to manufacture the displacement layer:

### A. Preparation of binder solution.

400 g of hydroxypropylmethylcellulose having an average molecular weight of 11,200 is added to a mixing vessel containing 5000 g of water. This mixture is stirred until the hydroxypropylmethylcellulose dissolves in water and a clear solution is formed. This solution is referred to as the binder solution.

### B. Preparation of osmotic layer granules.

First, 3912 g of hydroxyethylcellulose having an average molecular weight of 1,300,000 is placed into the bowl of a fluid bed granulator. Next, 400 g of hydroxypropylmethylcellulose having an average molecular weight of 11,200 is added to the bowl. Then, 3,200 g of sodium chloride and 80 g of black ferric oxide, which have been milled using a 21 mesh screen, is then placed into the bowl. The bowl is attached to the main body of the granulator and the granulation process is then initiated. Initially, the powder bed inside the bowl is fluidized for 3 minutes to obtain uniform mixing of the powders.. Next, the binder solution is sprayed at a rate of 80 g/min. During the spraying process, the process air flow is maintained at 400 cfm and the product temperature is maintained at 22°C. During the spraying operation, the solution is sprayed for 30 seconds followed by a shaking time of 10 seconds, during which time the powders adhering to the filterbags may be dislodged into the granulating chamber. At the end of the spraying operation, the granules are dried in the granulating chamber for a additional 10 to 15 minutes to obtain dry granulation. The process parameters may be adjusted to obtain a quality product. The granules are then passed through a fluid air mill with a 7 mesh screen for size reduction. The size-reduced granules are then placed into a blender. Then, 8 g of magnesium stearate (screened through a 40 mesh screen) and 4 g of powdered butylated hydroxy toluene (screened through a 60 mesh screen) is added to the granules and mixed together.

### C. Compression of the layers.

The methylphenidate granules-forming layer and the osmotic granules-forming layer are compressed together using an automated tablet compression machine capable of compressing the two layers together - longitudinally. First, 110 mg of methylphenidate granules (layer 1) is added into the die cavity of a 3/16" diameter tooling, tamped, and then 132 mg of the osmotic layer granulation (layer 2) is placed into the die and compressed together using 0.2 metric tons of pressure.

### D. Application of semipermeable membrane wall.

The semipermeable membrane wall forming composition is composed of 47.5% cellulose acetate 398 (having an acetyl content of 39.8), 47.5% cellulose acetate 320 (having an acetyl content of 32.0) and 5% polyethylene glycol having an average molecular weight of 3350. The semipermeable membrane composition is dissolved in a mixture of methylene chloride and methanol (the solvents are mixed together in a ratio of 80:20, wt:wt), such that the solids composition of the solution is 4%. The compressed systems are placed into a 24" coating pan and the coating solution is sprayed onto the tablets such that the solution is sprayed at a rate of 100 ml/min/gun. The product temperature is maintained at 35°C. The coating process is stopped when the desired amount of semipermeable membrane composition has been sprayed onto the compressed systems.

Next, one 30 mil (0.76 mm) orifice is drilled using a laser, on the drug layer side of the coated systems. The residual solvents remaining after the coating are removed by drying the systems at 45°C and 45% relative humidity in an oven for 48 hours. At the end of this drying cycle, the humidity is turned off and the systems are dried at 45°C for an additional 4 hours to complete the drying process.

A methylphenidate dosage form assembled as described contains 110 mg of drug containing layer 1, which is composed-of 12.8% methylphenidate hydrochloride, 50.2% to 80.2% poly(ethylene oxide) of average molecular weight 200,000, 0 to 30% sorbitol, 5% hydroxypropylmethylcellulose of average molecular weight 11,200, 2% magnesium stearate, and 0.05% butylated hydroxy toluene. The dosage form also contains 132 mg of layer 2, composed of 48.9% hydroxyethylcellulose of average molecular weight 1,300,000, 40% sodium chloride, 5% hydroxypropylmethylcellulose of average molecular weight 11,200, 1% red ferric oxide, 0.1 % magnesium stearate and 0.05% butylated hydroxy toluene. 42 mg of a semipermeable laminate composed of 47.5% cellulose acetate of acetyl content 39.8%, 47.5% cellulose acetate of acetyl content 32.0%, and 5% polyethylene glycol having an average molecular weight of 3350 is applied to the compressed bilayer system, and a 30 mil (0.76 mm) orifice is drilled on the drug layer side as the exit orifice. The final system is capable of delivering 14 mg of methylphenidate hydrochloride with an ascending release rate profile over a prolonged period of time.

### EXAMPLE 13

The manufacturing procedures described in the above examples are followed, except that in this example a dosage form is provided wherein a drug overcoat and an optional taste-masking coat is overcoated onto the semipermeable walled dosage form. In this example, the drug layer is composed of 14 mg methylphenidate hydrochloride, 27.5 mg sorbitol, 5.5 mg polyvinylpyrrolidone, 61 mg of poly(ethylene oxide) of average molecular weight 2,000,000, 2.2 mg of magnesium stearate and 0.055 mg of butylated hydroxy toluene. The second displacement layer is composed of 72 mg of poly(ethylene oxide) of average molecular weight 7,000,000, 53 mg of sodium chloride, 6.6 mg of polyvinylpyrrolidone, 1.3 mg of ferric oxide, 0.132 mg of magnesium stearate, and 0.066 mg of butylated hydroxy toluene. The semipermeable wall is composed of 20 mg of cellulose acetate of average acetyl content 39.8%, 20 mg of cellulose acetate of average acetyl content 32% and 2 mg of poly(ethylene oxide) of average molecular weight 4000.

The drug-containing overcoat is composed of 60% hydroxypropylmethylcellulose and 40% methylphenidate hydrochloride. The hydroxypropylmethylcellulose is added to water and mixed until a uniform solution results. Then, the methylphenidate hydrochloride is added to this solution and mixed such that a clear solution results. The final solution has a solids composition of 10%. The semipermeable walled dosage forms are placed in a coater and 10 mg of the drug overcoat is sprayed onto the semipermeable wall that surrounds the internal bilayer compressed tablet.

Next, the tablets are dried in the coating pan at 40°C for 10-15 minutes. For the taste masking coat, a suspension of Opadry® is prepared in water such that the solids content is 10%. Opadry® is a powder blend commercially available from Colorcon Inc., and is composed of hydroxypropylmethylcellulose, titanium dioxide, polyethylene glycol and polysorbate 80. The systems coated with the drug containing overcoat are placed into the coater, and the 9 mg of taste-masking coating solution is sprayed onto the systems. Next, the systems are dried in the coating pan at 40°C for 10-15 minutes to yield the operable dosage forms.

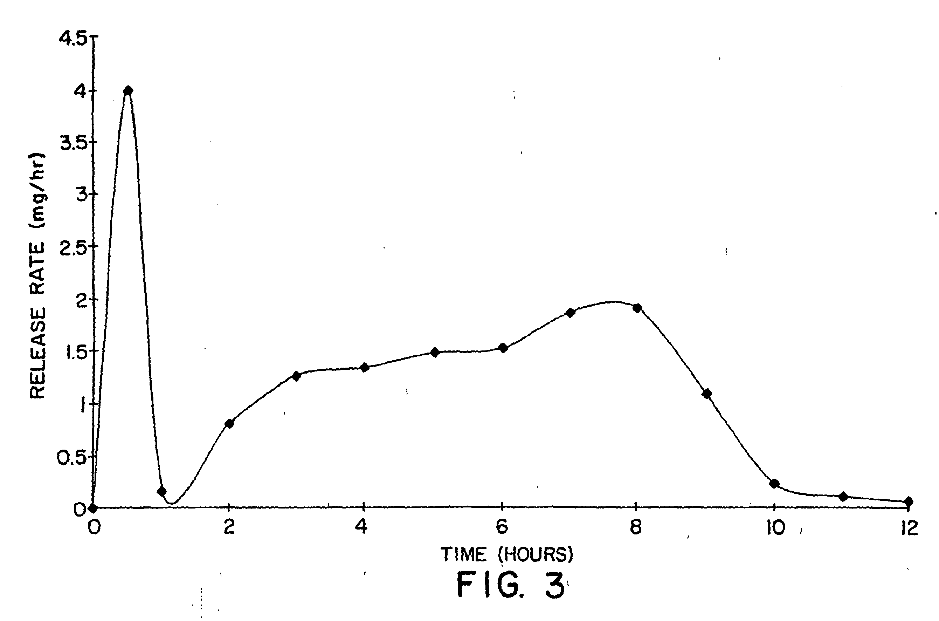

The accompanying Figure 3 represents the functionality of the dosage form of the example. The dosage form releases 4 mg of drug in the first half hour from the drug overcoat, followed by 0.41 mg in the next half hour, 1.05 mg in the second hour, 1.49 mg in the third hour, 1.57 mg in the fourth hour, 1.71 mg in the fifth hour, 1.75 mg in the sixth hour, 2.09 mg in the seventh hour, 2.14 mg in the eighth hour, 1.32 mg in the ninth hour and 0.48 mg in the tenth hour.

### EXAMPLE 14

The dosage form is manufactured as described in Example 13, wherein in the second displacement layer the poly(ethylene oxide) is replaced with 72 mg of hydroxyethylcellulose of 1,300,000 molecular weight.

### EXAMPLE 15

Dosage forms are provided according to the above examples wherein: (a) the dosage form comprises an overcoat of 8 mg of methylphenidate and an internal composition comprising 28 mg of methylphenidate; and (b) the dosage form comprises an overcoat of 12 mg of methylphenidate, and an internal composition comprising 42 mg of methylphenidate.

### EXAMPLES 16 TO 19

Dosage forms are provided by following the above disclosure and examples to provide dosage forms that deliver a dose of drug, for example, a central nervous system drug, in an ascending profile in the following therapeutic ranges: (a) a dosage form that delivers in the first hour 0 to 0.308 mg of drug, in the second hour 0.250 mg to 2 mg of drug, in the third hour 1 mg to 2.4 mg of drug, in the fourth hour 1.1 mg to 2.6 mg of drug, in the fifth hour 1.23 mg to 2.9 mg of drug, in the sixth hour 1.33 mg to 3.1 mg of drug, in the seventh hour 1.28 to 2.98 mg of drug, and in the eighth hour 0.76 mg to 1.76 mg of drug; (b) a dosage form exhibiting an ascending dose profile in the first hour 0 mg to 3.00 mg, in the second hour 2.75 mg to 10 mg, in the third hour 5 mg to 12 mg, in the fourth hour 5.5 mg to 13 mg, in the fifth hour 6.15 mg to 14.5 mg, in the sixth hour 6.65 to 15.5 mg, in the seventh hour 6.4 mg to 14.9 mg, and in the eighth hour 3.8 to 8.8 mg; (c) a dosage form comprising a drug ascending release rate program of 0 mg to 0.400 mg in the first hour, 0.376 mg to 1.81 mg in the second hour, 1.29 mg to 2.15 mg in the third hour, 1.38 mg to 2.3 mg in the fourth hour, 1.54 mg to 2.57 mg in the fifth hour, 1.66 mg to 2.76 mg in the sixth hour, 1.59 to 2.66 mg in the seventh hour, and 0.93 to 1.58 mg in the eighth hour; and (d) a dosage form that delivers an orally administrable drug in an ascending dose of 0 mg to 3.00 mg in the first hour, 2.45 mg to 9.05 mg in the second hour, 6.45 mg to 10.75 mg in the third hour, 6.9 mg to 11.5 mg in the fourth hour, 7.7 mg to 12.9 mg in the fifth hour, 8.3 mg to 13.8 mg in the sixth hour, 7.95 mg to 133 mg in the seventh hour, and 4.65 mg to 7.9 mg in the eighth hour.

### EXAMPLES 20 TO 21

The procedures set forth in the above examples are followed with the manufacture as described above, except in this invention, a first dosage form is provided wherein the drug layer comprises 28 mg of methylphenidate hydrochloride and a second dosage form is provided where the drug layer comprises 42 mg of methylphenidate hydrochloride.

### DISCLOSURE OF METHOD OF USING THE INVENTION

The invention pertains further to the use of the dosage form for the manufacture of a medicament for delivering an ascending dose over time to a warm-blooded animal in need of therapy. The invention facilitates: (a) a method for delivering a dose of drug in an increasing rate to a patient, wherein the method comprises administering orally to the patient a drug in an increasing rate per hour overtime, to provide the dose of drug to the patient; (b) a method for delivering a dose of drug in an increasing dose of drug to a patient, wherein the method comprises delivering orally to the patient an orally administrable drug, in an increasing milligram dose per hour over twenty-four hours, to deliver the dose of drug; (c) a method for delivering a drug to a patient from a dosage form over time, wherein the method comprises admitting orally into the patient a dosage form comprising 240 nanograms to 700 milligrams of drug that is delivered in an increasing dose over time; and (d) a method comprising the steps of: (A) admitting into a patient a dosage form comprising: (1) a wall that surrounds a compartment; the wall comprising a semipermeable composition permeable to the passage of fluid, including aqueous-biological fluid of the gastrointestinal tract, and impermeable to the passage of drug; (2) a bilayer in the compartment comprising a first layer comprising a dose of drug, a second layer comprising an osmopolymer and an osmagent, and for imbibing and absorbing fluid for pushing the first layer from the dosage form and thereby providing an increased dose per unit time over time; and (3) at least one exit in the wall communicating with the first layer; (B) imbibing fluid through the semipermeable wall at a rate determined by the permeability of the semipermeable wall and the osmotic gradient across the semipermeable wall causing the second layer to expand and swell; and (C) deliver the drug from the first layer through the exit passageway to provide an ascending, increasing dose of drug to the patient.

In summary, it will be appreciated the present invention contributes to the art an unexpected dosage form that possesses the practical utility for administering a sustained and increasing dose of drug at a dosage-metered release rate over time.

## Claims

1. An osmotic dosage form for delivering a central nervous system acting drug to a patient at an ascending release rate for 2 to 24 hours, said osmotic dosage form comprising:
(a) a compressed bi-layer core containing 10 ng to 700 mg of said drug in a first layer and a combination of osmopolymer and osmagent in a second layer, wherein said first layer further comprises up to 200 mg sorbitol, and said combination of osmopolymer and osmagent comprises 60 to 95% of said second layer;
(b) a semipermeable wall surrounding said bi-layer core; and
(c) an exit in said wall communicating with the first layer for delivering the drug;
wherein said dosage form is a dosage form tablet which comprises a length of 5 mm to 28 mm and a width of 2.50 mm to 10 mm.

2. The dosage form as claimed in claim 1 wherein said CNS-acting drug is selected from the group consisting of methylphenidate, threo-methylphenidate, amphetamine, dextroamphetamine, methamphetamine, phenylisoprapylamine and pemoline.

3. The dosage form as claimed in claim 1 or claim 2 wherein said osmotic dosage form is provided with an outer coating comprising an additional immediate-release dose of said drug.

4. Use of a dosage form as claimed in any one of claims 1 to 3 for the manufacture of a medicament for use in a method of treatment in which said drug is released at an ascending release rate.

## Patentansprüche

1. Osmotische Dosierungsform zur Darreichung eines auf das zentrale Nervensystem einwirkenden Wirkstoffs an einen Patienten mit ansteigender Freisetzungsrate über 2 bis 24 Stunden, wobei die osmotische Dosierungsform umfasst:
(a) einen komprimierten zweilagigen Kern, der 10 ng bis 700 mg des Wirkstoffs in einer ersten Lage und eine Kombination eines Osmopolymers und eines Osmagents in einer zweiten Lage, wobei die erste Lage außerdem bis zu 200 mg Sorbitol umfasst und die Kombination von Osmopolymer und Osmagent 60 bis 95 % der zweiten Lage ausmacht;
(b) eine semipermeable Wand, die den zweilagigen Kern umgibt; und (c) einen Auslass in der Wand, der mit der ersten Lage zur Abgabe des Wirkstoffs in Verbindung steht;
wobei es sich bei der Dosierungsform um eine Dosierungsform in Gestalt einer Tablette handelt, die eine Länge von 5 mm bis 28 mm und eine Breite von 2,50 mm bis 10 mm aufweist.

2. Dosierungsform nach Anspruch 1, wobei der ZNS-wirksame Wirkstoff unter Methylphenidat, threo-Methylphenidat, Amphetamin, Dextroamphetamin, Methamphetamin, Phenylisopropylamin und Pemolin ausgewählt ist.

3. Dosierungsform nach Anspruch 1 oder 2, wobei die osmotische Dosierungsform mit einer äußeren Umhüllung versehen ist, die eine zusätzliche unmittelbar freizusetzende Dosis des Wirkstoffs umfasst.

4. Verwendung einer Dosierungsform nach einem der Ansprüche 1 bis 3 zur Herstellung eines Arzneimittels zur Anwendung in einem Behandlungsverfahren, wobei der Wirkstoff mit ansteigender Freisetzungsrate freigesetzt wird.

## Revendications

1. Forme galénique osmotique pour administrer un médicament agissant sur le système nerveux central à un patient à une vitesse de libération croissante pendant 2 à 24 heures, la forme galénique osmotique comprenant :
(a) un noyau comprimé à deux couches contenant de 10 ng à 700 mg du médicament dans une première couche et une combinaison d'un osmopolymère et d'un osmoagent dans une deuxième couche, la première couche comprenant, en outre, jusqu'à 200 mg de sorbitol et la combinaison de l'osmopolymère et de l'osmoagent représentant de 60 à 95 % de la deuxième couche ;
(b) une paroi semi-perméable entourant le noyau à deux couches ; et
(c) une sortie ménagée dans la paroi communiquant avec la première couche pour l'administration du médicament ;
dans laquelle la forme galénique est un comprimé pour forme galénique qui a une longueur de 5 mm à 28 mm et une largeur de 2,50 mm et 10 mm.

2. Forme galénique suivant la revendication 1, dans laquelle le médicament agissant sur le SNC est choisi dans le groupe consistant en le phénidate de méthyle, le phénidate de thréométhyle, l'amphétamine, la dextroamphétamine, la méthamphétamine, la phénylisopropylamine et la pémoline.

3. Forme galénique suivant la revendication 1 ou la revendication 2, dans laquelle la forme galénique osmotique est munie d'un enrobage extérieur comprenant une dose supplémentaire à libération immédiate du médicament.

4. Utilisation d'une forme galénique tel que revendiquée à l'un quelconque des revendications 1 à 3 pour la fabrication d'un médicament à utiliser dans un procédé de traitement dans lequel le médicament est libéré à une vitesse de libération croissante.
